# EUROPEAN PATENT APPLICATION

(11) **EP 4 290 527 A1**
(43) Date of publication of application: **13.12.2023**
(21) Application number: 22771710.5
(22) Date of filing: 14.03.2022
(51) Int. Cl.: G16H 30/40, G16H 50/20, G16H 30/20, G06N 3/08, G06T 11/60, G06T 7/11

(54) **BLADDER LESION DIAGNOSIS METHOD USING NEURAL NETWORK, AND SYSTEM THEREOF**

(30) Priority: 15.03.2021 KR 20210033189
(71) Applicant: Deep Bio Inc., Seoul 08380 (KR)
(72) Inventor: PAIK, In Young, Seoul 03680 (KR); KWAK, Tae Yeong, Seoul 05119 (KR); KIM, Sun Woo, Seongnam-si, Gyeonggi-do 13599 (KR)
(74) Representative: Flügel Preissner Schober Seidel
(86) International application number: PCT/KR2022/003546
(87) International publication number: WO 2022/197044

(57) **Abstract**

Disclosed are a bladder lesion diagnosis method using a neural network, and a system thereof. The bladder lesion diagnosis method using a neural network comprises the steps of: receiving a unit pathological image by a bladder lesion diagnosis system; inputting, by the bladder lesion diagnosis system, the unit pathological image into a first neural network to obtain the diagnosis result of a first bladder lesion among a plurality of bladder lesions in the unit pathological image; and inputting, by the bladder lesion diagnosis system, the unit pathological image into a second neural network to obtain the diagnosis result of a second bladder lesion, other than the first bladder lesion, among the plurality of bladder lesions in the unit pathological image. The first neural network is a neural network learned through a plurality of pieces of first learning data obtained by annotating a lesion region in which the first bladder lesion is expressed, and the second neural network is a neural network learned through a plurality of pieces of second learning data obtained by annotating the type of expressed lesion and indicating whether at least one of the second bladder lesions is expressed, wherein the second learning data is data obtained without annotation of the lesion region.

## Description

### Technical Field

The present disclosure relates to a bladder lesion diagnosis method using a neural network and a system thereof, and more particularly, to a method capable of effectively training a diagnosis system capable of diagnosing a plurality of bladder lesions using pathological images based on living tissue of the bladder and using the same for diagnosis and a system thereof.

### Background Art

Bladder cancer is one of the most common types of cancer and has a high chance of recurrence. As a method for diagnosing and locally treating bladder cancer, transurethral resection of bladder (TURB) is mainly used.

In general, tissue specimens obtained through TURB are pathologically read for diagnosis such as to confirm bladder cancer and determine the severity.

At this time, the tissue specimens obtained through TURB may be used not only to diagnose bladder cancer but also to diagnose various lesions related to the bladder.

For example, there are many types of lesions that may be identified in the tissue specimen obtained through TURB, and they may be largely divided into invasive cancer lesions and non-invasive cancer lesions.

In addition, non-invasive lesions include cancer lesions such as low/high grade noninvasive papillary urothelial carcinoma, and urothelial carcinoma in situ (CIS), and proliferative lesions such as papillary urothelial neoplasm of low malignant potential (PUNLMP), urothelial proliferation of unknown malignant potential (UPUMP), urothelial papilloma, inverted urothelial papilloma, and urothelial dysplasia.

In addition, it may be very costly and inefficient to exclusively diagnose these various bladder lesions by pathologists.

Therefore, there is an increasing demand for a machine learning model (neural network) capable of diagnosing (detecting) bladder cancer lesions by analyzing a pathological image created by scanning a pathology slide of a tissue specimen obtained through TURB with a scanner.

In particular, since the size of the tissue specimen of TURB is relatively large, examining specimens in the form of glass slides with an optical microscope or reading specimens in the form of pathological images on a monitor to find lesions of bladder cancer and determine the type may be tiring and difficult for pathologists, thereby increasing the demand.

In addition, technical ideas are required to effectively build these machine learning models.

### [Prior Art Document]

### - Non-Patent Document

(Non-Patent Document 1) Non-patent document: (Antoni2016) Antoni, S. et al., Bladder Cancer Incidence and Mortality: A Global Overview and Recent Trends.

### Disclosure of the Invention

### Technical Goals

A technical object to be achieved by the present disclosure is to provide a technical idea to implement a neural network-based diagnosis system that may additionally diagnose various bladder lesions through tissue specimens obtained through TURB performed as a diagnosis and local treatment method for bladder cancer.

In addition, an object is to provide a technical idea that may provide the efficiency of constructing a diagnosis system, by constructing a neural network using training data annotated with the lesion region only for bladder lesions that must be learned by annotating the lesion region limitedly, and constructing a neural network using training data annotated only with the type of lesion for the rest of the bladder lesions in the diagnosis system.

### Technical Solutions

A bladder lesion diagnosis method using a neural network for achieving the above technical objects includes receiving, by a bladder lesion diagnosis system, a unit pathological image as input, inputting, by the bladder lesion diagnosis system, the unit pathological image to a first neural network to obtain a diagnosis result of a first bladder lesion among a plurality of bladder lesions in the unit pathological image, and inputting, by the bladder lesion diagnosis system, the unit pathological image to a second neural network to obtain a diagnosis result of a second bladder lesion excluding the first bladder lesion among the plurality of bladder lesions in the unit pathological image, wherein the first neural network is a neural network trained through a plurality of first training data annotated with a lesion region in which the first bladder lesion is expressed, and the second neural network is a neural network trained through a plurality of second training data annotated with an expressed lesion type indicating whether at least one of the second bladder lesion is expressed, wherein the second training data is data for which annotation for the lesion region is not performed.

The unit pathological image may be a patch image obtained by dividing a pathological image corresponding to a tissue specimen obtained through transurenthral resection of bladder (TURB) into a predetermined size.

The first bladder lesion may include a urothelial carcinoma in situ (CIS) lesion.

The second bladder lesion may include at least one of an invasive urothelial carcinoma, a low/high grade noninvasive papillary urothelial carcinoma lesion, a papillary urothelial neoplasm of low malignant potential (PUNLMP) lesion, a urothelial proliferation of unknown malignant potential (UPUMP) lesion, a urothelial papilloma lesion, an inverted urothelial papilloma lesion, and a urothelial dysplasia lesion.

A bladder lesion diagnosis method using a neural network for achieving the above technical objects includes receiving, by a bladder lesion diagnosis system, a unit pathological image as input, inputting, by the bladder lesion diagnosis system, the unit pathological image to a second neural network to obtain a diagnosis result of a second bladder lesion excluding a first bladder lesion among a plurality of bladder lesions in the unit pathological image, and inputting, by the bladder lesion diagnosis system, the unit pathological image to a first neural network to obtain a diagnosis result of the first bladder lesion among the plurality of bladder lesions in the unit pathological image, wherein the first neural network is a neural network trained through a plurality of first training data annotated with a lesion region in which the first bladder lesion is expressed, and the second neural network is a neural network trained through a plurality of second training data annotated with an expressed lesion type indicating whether at least one of the second bladder lesion is expressed, wherein the second training data is data for which annotation for the lesion region is not performed.

The method may be implemented by a computer program stored in a non-transitory computer readable recording medium.

According to another aspect of the present disclosure, a bladder lesion diagnosis system using a neural network includes a processor, and a memory in which a program executed by the processor is recorded, wherein the processor is configured to drive the program to input a unit pathological image to a first neural network to obtain a diagnosis result of a first bladder lesion among a plurality of bladder lesions in the unit pathological image, and input the unit pathological image to a second neural network to obtain a diagnosis result of a second bladder lesion excluding the first bladder lesion among the plurality of bladder lesions in the unit pathological image, wherein the first neural network is a neural network trained through a plurality of first training data annotated with a lesion region in which the first bladder lesion is expressed, and the second neural network is a neural network trained through a plurality of second training data annotated with an expressed lesion type indicating whether at least one of the second bladder lesion is expressed, wherein the second training data is data for which annotation for the lesion region is not performed.

### Advantageous Effects

According to the technical idea of the present disclosure, there is an effect of diagnosing not only bladder cancer but also various bladder lesions through TURB through a neural network-based diagnosis system that may additionally diagnose various bladder lesions through tissue specimens obtained through TURB performed as a diagnosis and local treatment method for bladder cancer.

In addition, in constructing a diagnosis system, by constructing a neural network using training data annotated with the lesion region only for bladder lesions that must be learned by annotating the lesion region limitedly, and constructing a neural network using training data annotated only with the type of lesion for the rest of the bladder lesions, there is an effect of effectively constructing a diagnosis system.

### Brief Description of Drawings

In order to more fully understand the drawings cited in the detailed description of the present disclosure, a brief description of each drawing is provided.
FIG. 1 is a diagram for explaining a schematic system for implementing a bladder lesion diagnosis method using a neural network in accordance with a technical idea of the present disclosure.
FIG. 2 is a diagram for explaining a logical configuration of a bladder lesion diagnosis system using a neural network in accordance with a technical idea of the present disclosure.
FIG. 3 is a diagram for explaining a physical configuration of a bladder lesion diagnosis system using a neural network in accordance with a technical idea of the present disclosure.
FIG. 4 is a diagram for explaining a concept of a bladder lesion diagnosis method using a neural network in accordance with a technical idea of the present disclosure.
FIG. 5 is a flowchart for explaining a bladder lesion diagnosis method using a neural network in accordance with a technical idea of the present disclosure.

### Best Mode for Carrying Out the Invention

In order to fully understand the present disclosure and the advantages in operation of the present disclosure and the objects achieved by the practice of the present disclosure, reference should be made to the accompanying drawings illustrating preferred embodiments of the present disclosure and the contents described in the accompanying drawings.

In addition, in the present specification, when one component 'transmits' data to another component, it means that the component may directly transmit the data to the other component, or may transmit the data to the other component through at least one other component. Conversely, when one component 'directly transmits' data to another component, it means that the data is transmitted from the component to the other component without going through the other component.

Hereinafter, the present disclosure will be described in detail by describing preferred embodiments of the present disclosure with reference to the accompanying drawings. Like reference numerals in each drawing indicate like elements.

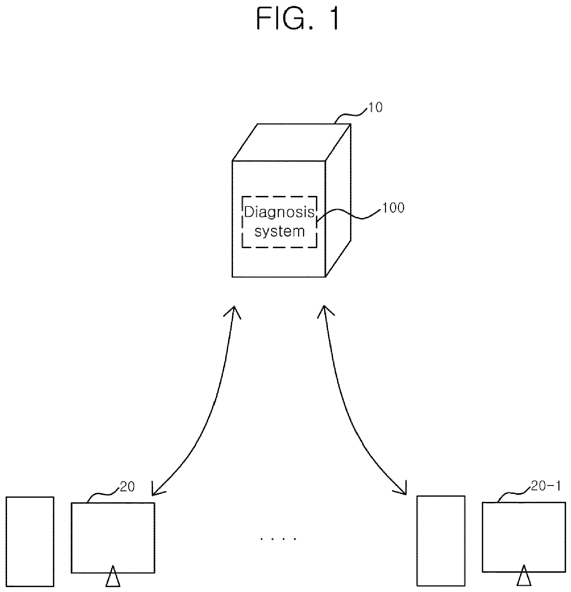

FIG. 1 is a diagram for explaining a schematic system for implementing a bladder lesion diagnosis method using a neural network in accordance with a technical idea of the present disclosure.

Referring to FIG. 1, a bladder lesion diagnosis method using a neural network in accordance with a technical idea of the present disclosure (hereinafter, diagnosis system 100) may be installed in a server 10 to implement the technical idea of the present disclosure. An average expert in the technical field of the present disclosure may easily infer that the server 10 refers to a data processing device having an arithmetic capability for implementing the technical idea of the present disclosure, and in general, may be defined as any device capable of performing a specific service, such as a personal computer, a portable terminal, or the like, as well as data processing devices accessible to clients over a network.

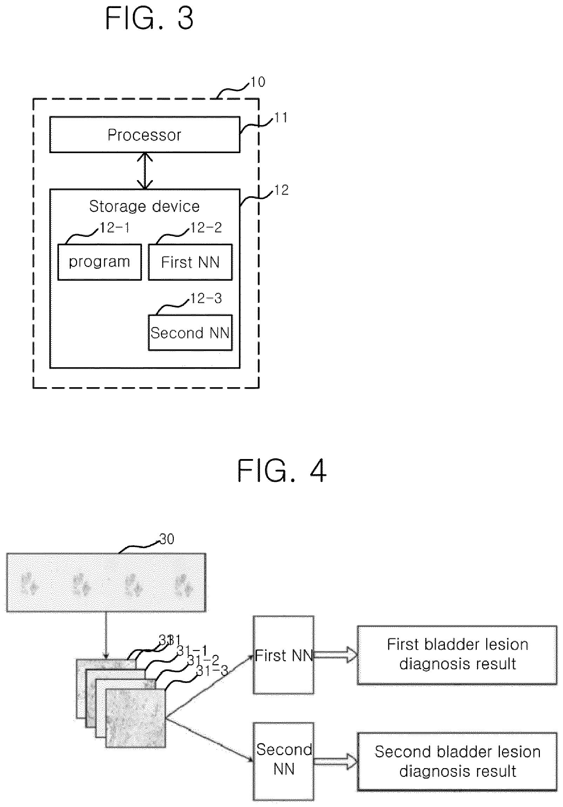

The server 10 may include a processor 11 and a storage device 12 as shown in FIG. 3. The processor 11 may refer to an arithmetic device capable of driving the program 12-1 for implementing the technical idea of the present disclosure, and the processor 11 may perform diagnosis using the program 12-1 and a plurality of neural networks 12-2 and 12-3 defined by the technical idea of the present disclosure.

The processor 110 may refer to an arithmetic device capable of executing a predetermined program (software code) and may be named by various names such as an embodiment of the data processing device or a vendor mobile processor, microprocessor, CPU, single processor, multiprocessor, GPU, etc., and may be implemented as one or more processors.

An average expert in the technical field of the present disclosure may easily infer that the processor 110 may drive the program and perform data processing necessary for the technical idea of the present disclosure.

The storage device 120 may refer to a device in which a program for implementing the technical idea of the present disclosure is stored/installed. Depending on an embodiment, the storage device 120 may be divided into a plurality of different physical devices, and a part of the storage device 120 may exist inside the processor 110 depending on an embodiment. The storage device 120 may be implemented as a hard disk, a GPU, a solid state disk (SSD), an optical disk, a random access memory (RAM), and/or other various types of storage media, depending on an embodiment, and may be detachably implemented in the storage device 120 if necessary.

In the present specification, when the diagnosis system 100 performs diagnosis, it may mean that the processor 11 drives the program 12-1 to output whether or not at least one of a plurality of bladder lesions is expressed and/or an expression region through the neural networks 12-2 and 12-3.

The expression region of a predetermined bladder lesion may be determined in units of pixels, and to this end, it has been known that a neural network for determining whether or not included in a predetermined disease expression region for each pixel may be trained and utilized, so a detailed description will be omitted in the present specification.

The storage device 12 may refer to a data storage means capable of storing the program 12-1 and the neural networks 12-2 and 12-3, and may be implemented as a plurality of storage means depending on an embodiment. In addition, the storage device 12 may refer to not only the main storage device included in the server 10, but also a temporary storage device or memory that may be included in the processor 11.

Although the diagnosis system 100 is shown as being implemented as any one physical device in FIG. 1 or 3, an average expert in the technical field of the present disclosure may easily infer that a plurality of physical devices may be organically combined as needed to implement the diagnosis system 100 according to the technical idea of the present disclosure.

In the present specification, that the diagnosis system 100 performs diagnosis may refer to a series of processes of receiving a unit pathological image and outputting output data defined herein. The output data may be data indicating the type of lesion expressed when one or a plurality among the plurality of bladder lesions are expressed in the unit pathological image as described above. Alternatively, not only the type of lesion but also information indicating the region in which the lesion was expressed (e.g., whether included in the lesion region for each pixel) may be further included in the output data.

The unit pathological image may be a patch image obtained by dividing a slide image of a tissue specimen obtained through TURB is divided into a predetermined size. The size of such patch images may be appropriately determined as needed.

As described above, various bladder lesions may be diagnosed through tissue specimens obtained through TURB, but it may take a lot of time and cost for a pathologist to diagnose them while individually checking slide images.

Therefore, a machine learning model capable of diagnosing various bladder lesions through tissue specimens obtained through TURB, i.e., at least one trained neural network, may be required.

In general, the method for obtaining the most accurate results in developing such a machine learning model is to obtain a large amount of training data in which the types and regions of lesions expressed for each pathological image (a slide image or a patch image obtained by dividing the slide image) of a tissue specimen are individually annotated, and to train the obtained large amount of training data through a neural network. In other words, by training a machine learning model in a supervised-learning method, a neural network that outputs (diagnoses) whether bladder lesions are expressed on the pathological image and, if so, what type of bladder lesions is expressed may be trained. In addition, as described above, if necessary, the neural network may be trained to output even the region of the expressed bladder lesion.

However, since tissue specimens obtained through TURB and their pathological images themselves are relatively large, and the types of bladder lesions are diverse, it is very difficult for pathologists to annotate the types and lesion regions of each type of lesion individually, and accordingly, there is a problem that constructing a large amount of training data is also very costly and time-consuming.

In particular, compared to annotating only the types of lesions expressed on the pathological image (unit pathological image), there is a problem in that relatively large resources are required to annotate the expressed lesion region on the pathological image, and in order to solve this problem, a method of annotating only the type of lesion, which lesion is are expressed in the corresponding pathological image, without annotating the lesion region in the pathological image may be considered.

In addition, it is possible to develop a machine learning model that discriminates the lesion region and/or type using predetermined supervised-learning methods only with the lesion type information present in the pathological image without annotation information on the lesion region, and at this time, each pathological image is annotated with a number of lesion types that may be identified in the image, and as supervised-learning methods using this, semi/unsupervised learning (Xie2019) using consistency loss, noisy label framework (Li2020), reject option (Geifman2019), (min/max/attention) multiple-instance learning (Ilse2018) and the like may be used.

In addition, the present applicant has established a diagnosis system for diagnosing various bladder lesions by training a neural network by annotating only the types of lesions expressed in unit pathological images in this way, and has tested its performance.

However, it was confirmed that among the various bladder lesions (e.g., low/high grade noninvasive papillary urothelial carcinoma, urothelial carcinoma in situ (CIS), papillary urothelial neoplasm of low malignant potential (PUNLMP), urothelial proliferation of unknown malignant potential (UPLTMP), urothelial papilloma, inverted urothelial papilloma, urothelial dysplasia, etc.), in most cases, even when trained with training data annotated only with such lesion type, the type of lesion and/or the lesion region may be diagnosed well.

However, as described above, it was confirmed that CIS-type lesions have characteristics that they are difficult to diagnose with a neural network trained with training data annotated only with the type of lesion on a unit pathological image. This may be because CIS-type lesions are often classified according to cell types rather than tissue types, and thus have characteristics that must be discriminated in a different way from other types of lesions.

Therefore, the present disclosure may provide a technical idea for solving this problem.

According to the technical idea of the present disclosure, various bladder lesions are classified into a first bladder lesion and a second bladder lesion, and for lesions classified as the first bladder lesion, a neural network trained with a plurality of first training data in which both lesion types and lesion regions are annotated, i.e., the first neural network is constructed, and diagnosis may be performed through the first neural network.

And for the remaining bladder lesions, i.e., the lesions classified as the second bladder lesion, a neural network trained with a plurality of second training data annotated only with the lesion type i.e., a second neural network is constructed, and diagnosis may be performed through the second neural network. In this case, the second training data may be training data on which annotation is not performed on the aforementioned lesion region.

Through this, even when training by annotating only the type of lesion among various bladder lesions, for lesions with good diagnostic performance, only the types of lesions are annotated and trained, which has the effect of making training data construction as efficient as possible.

In addition, by annotating the lesion type and lesion region only for limited lesions (e.g., CIS lesions) in which diagnostic performance is exhibited above a certain level only when the lesion region is additionally annotated and trained due to the nature of the lesion, there is an effect of guaranteeing diagnostic performance for bladder lesions as a whole.

When the diagnosis system 100 is implemented by being included in a predetermined server 10, the diagnosis system 100 may perform communication with at least one client (e.g., 20, 20-1) accessible to the server 10. In this case, the client (e.g., 20 or 20-1) may transmit a pathological image or unit pathological image to the diagnosis system 100, and the diagnosis system 100 may perform diagnosis according to the technical idea of the present disclosure on the transmitted pathological image or unit pathological image. In addition, the diagnosis result may be transmitted to the client (e.g., 20, 20-1).

Depending on the embodiment, the server 10 itself may be equipped with an interface for receiving the pathological image or unit pathology image.

The diagnosis system 100 may perform diagnosis on various bladder lesions using a neural network according to the technical idea of the present disclosure. A process of training the neural network may be first performed in order to perform such a diagnosis.

Accordingly, the diagnosis system 100 may be a system that performs diagnosis by receiving a neural network trained according to the technical idea of the present disclosure and a program for performing diagnosis using the neural network from the outside, or may be a system that even performs training of the neural network. In addition, the diagnosis system 100 may be implemented as a dedicated device manufactured to implement the technical idea of the present disclosure, not a general-purpose data processing device, and in this case, a means for scanning a pathological image may be further provided.

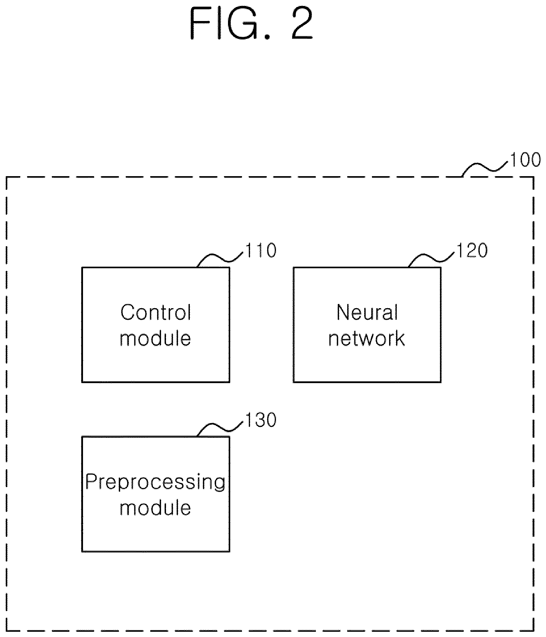

The diagnosis system 100 for implementing this technical idea may logically have a configuration as shown in FIG. 2.

FIG. 2 is a diagram for explaining a logical configuration of a disease diagnosis system using a neural network in accordance with an embodiment of the present disclosure.

Referring to FIG. 2, the diagnosis system 100 includes a control module 110 and a neural network module 120 in which a neural network is stored. In addition, the diagnosis system 100 may further include a preprocessing module 130.

The diagnosis system 100 may refer to a logical configuration including hardware resources and/or software required to implement the technical idea of the present disclosure, and does not necessarily mean one physical component or one device. In other words, the diagnosis system 100 may refer to a logical combination of hardware and/or software provided to implement the technical idea of the present disclosure, and if necessary, it may be implemented as a set of logical components to implement the technical idea of the present disclosure by being installed in devices spaced apart from each other and performing respective functions. In addition, the diagnosis system 100 may refer to a set of components separately implemented for each function or role to implement the technical idea of the present disclosure. For example, each of the control module 110, the neural network module 120, and/or the preprocessing module 130 may be located in different physical devices or in the same physical device. In addition, depending on the embodiment, a combination of software and/or hardware constituting each of the control module 110, the neural network module 120, and/or the preprocessing module 130 may also be located in different physical devices, and components located in different physical devices may be organically combined with each other to implement each of the modules.

In addition, in the present specification, a module may refer to a functional and structural combination of hardware for implementing the technical idea of the present disclosure and software for driving the hardware. For example, it may be easily inferred to an average expert in the technical field of the present disclosure that the module may refer to a logical unit of a predetermined code and a hardware resource for executing the predetermined code, and does not necessarily mean physically connected codes or one type of hardware.

The control module 110 may control other components (e.g., the neural network module 120 and/or the preprocessing module 130, etc.) included in the diagnosis system 100 to implement the technical idea of the present disclosure.

In addition, the control module 110 may perform diagnosis according to the technical idea of the present disclosure using the neural network stored in the neural network module 120.

The neural network module 120 may store a plurality of neural networks as described above. The neural network may refer to a set of information representing a series of design matters defining the neural network. An average expert in the technical field of the present disclosure may easily infer that in the present specification, the neural network may be a convolutional neural network, but various types of neural networks capable of performing a diagnosis by well extracting features based on the type and/or region of the lesion annotated on the pathological image may be used.

As is well known, the convolutional neural network may include an input layer, a plurality of hidden layers, and an output layer. Each of the plurality of hidden layers may include a convolutional layer and a pooling layer (or subsampling layer). In addition, recently, convolutional neural networks may be designed to include a normalization layer such as a batch normalization (BN) layer.

The convolutional neural network may be defined by a function, filter, stride, weight factor, etc., for defining each of these layers. In addition, the output layer may be defined as a fully connected FeedForward layer.

The design matters for each layer constituting the convolutional neural network are widely known. For example, known functions may be used for each of the number of layers to be included in the plurality of layers, the convolutional function, the pooling function, and the activation function for defining the plurality of layers, or separately defined functions may be used to implement the technical idea of the present disclosure.

An example of the convolutional function is a discrete convolution sum. As an example of the pooling function, max pooling, average pooling, and the like may be used. An example of the activation function may be a sigmoid, a tangent hyperbolic (tanh), a rectified linear unit (ReLU), a swish, an exponential linear unit (ELU), and the like.

When the design matters of the convolutional neural network are defined, the convolutional neural network with the design matters defined may be stored in a storage device. In addition, when the convolutional neural network is trained, weight factors corresponding to each layer may be specified.

In other words, training of the convolutional neural network may refer to a process in which weight factors of each layer are determined. When the convolutional neural network is trained, the trained convolutional neural network may receive input data through an input layer and output output data through a predefined output layer.

The neural network according to an embodiment of the present disclosure may be defined by selecting any one or a plurality of well-known design matters as described above, or an independent design matter may be defined for the neural network.

The control module 110 may sequentially input a unit pathological image, i.e., a pathological image to be diagnosed, to the plurality of neural networks stored in the neural network module 120. As described above, the unit pathological image may be a patch image obtained by separating a slide image into a predetermined size.

In this case, the plurality of neural networks may include a first neural network and a second neural network. In addition, the first neural network and the second neural network are separately trained and constructed, and information annotated in the training data used at this time may also be different. The types of deep learning models used in the first neural network and the second neural network may also be different from each other.

As described above, the first neural network may be a neural network for diagnosing the first bladder lesion. The plurality of first training data used for training of the first neural network may be training data, i.e., information in which lesion regions are annotated for each pathological image. When there are multiple types of first bladder lesions, it is necessary to separately annotate the types of lesions.

Such first bladder lesion may include a carcinoma in situ (CIS) lesion, and other lesions may be further included in the first bladder lesion as needed.

The second neural network may be a neural network for diagnosing a second bladder lesion as described above. The plurality of second training data used for training of the second neural network may be training data, i.e., information in which only the type of lesion is annotated without annotating the region of bladder lesion for each pathological image.

Such a second bladder lesion may include invasive urothelial carcinoma, low/high grade noninvasive papillary urothelial carcinoma, papillary urothelial neoplasm of low malignant potential (PUNLMP), urothelial proliferation of unknown malignant potential (UPUMP), urothelial papilloma, inverted urothelial papilloma, urothelial dysplasia, and other lesions may be further included in the second bladder lesion as needed.

The control module 110 may first perform a diagnosis on the first bladder lesion by inputting the unit pathological image to the first neural network. In addition, diagnosis on the second bladder lesion may be performed by inputting the unit pathological image into the second neural network. The unit pathological image may be first input to the second neural network and then the unit pathological image may be input to the first neural network, and diagnosis may be performed in parallel by being input at the same time.

The preprocessing module 130 may perform preprocessing of the pathological image needed before performing diagnosis using the neural network. For example, an average expert in the technical field of the present disclosure may easily infer that the preprocessing of the pathological image may include dividing the pathological image into patches of a predefined size, and that appropriate image processing may be performed in a manner suitable for each of the neural networks as needed.

FIG. 4 is a diagram for explaining a concept of a bladder lesion diagnosis method using a neural network in accordance with a technical idea of the present disclosure.

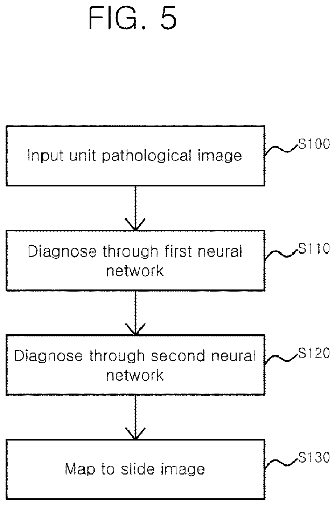

In addition, FIG. 5 is a flowchart for explaining a bladder lesion diagnosis method using a neural network in accordance with a technical idea of the present disclosure.

Referring to FIGS. 4 and 5, the diagnosis system 100 may receive a plurality of unit pathological images 31, 31-1, 31-2, 31-3, etc. to be diagnosed (S100).

The unit pathological images 31, 31-1, 31-2, 31-3, etc. may be patch images in which at least one pathological image (e.g., slide image, 30) generated from a tissue specimen obtained through TURB is divided into a predetermined size.

Each of the unit pathological images 31, 31-1, 31-2, 31-3, etc. may be input to both the first neural network and the second neural network (S 110, S120). At this time, each of the unit pathological images 31, 31-1, 31-2, 31-3, etc. may be first input to the first neural network to diagnose the first bladder lesion, and then input to the second neural network to diagnose the second bladder lesion, the order may be changed, or the diagnosis may be performed simultaneously.

For each of the unit pathological images 31, 31-1, 31-2, 31-3, etc., when a diagnosis is performed on the lesion type and/or region of the first bladder lesion through the first neural network and the lesion type and/or region of the second bladder lesion through the second neural network, the diagnosis result of each of the unit pathological images 31, 31-1, 31-2, 31-3, etc. may be mapped to a slide image (S 130).

Then, the diagnosis result for the slide image, i.e., information indicating the lesion region and/or type in the slide image may be obtained.

After all, according to the technical idea of the present disclosure, it is possible to implement a neural network-based diagnosis system that may additionally diagnose various bladder lesions through tissue specimens obtained through TURB performed as a diagnosis and local treatment method for bladder cancer, and in such a diagnosis system, by constructing a neural network using training data annotated with the lesion region only for bladder lesions that must be learned by annotating the lesion region limitedly, and constructing a neural network using training data annotated only with the type of lesion for the rest of the bladder lesions, there is an effect of effectively constructing a diagnosis system.

The bladder lesion diagnosis method using a neural network according to an embodiment of the present disclosure may be implemented as computer readable code on a computer readable recording medium. A computer-readable recording medium includes all types of recording devices in which data that may be read by a computer system is stored. Examples of computer-readable recording media include ROM, RAM, CD-ROM, magnetic tape, hard disk, floppy disk, and optical data storage devices. In addition, the computer-readable recording medium is distributed in computer systems connected through a network, so that computer-readable codes may be stored and executed in a distributed manner. In addition, functional programs, codes, and code segments for implementing the present disclosure may be easily inferred by programmers in the art to which the present disclosure belongs.

Although the present disclosure has been described with reference to an embodiment shown in the drawings, this is merely exemplary, and those skilled in the art will understand that various modifications and equivalent other embodiments are possible therefrom. Therefore, the true technical protection scope of the present disclosure should be determined by the technical idea of the attached claims.

### Industrial Applicability

The present disclosure may be used for a bladder lesion diagnosis method using a neural network and a system thereof.

## Claims

1. A bladder lesion diagnosis method using a neural network, comprising:
receiving, by a bladder lesion diagnosis system, a unit pathological image as input;
inputting, by the bladder lesion diagnosis system, the unit pathological image to a first neural network to obtain a diagnosis result of a first bladder lesion among a plurality of bladder lesions in the unit pathological image; and
inputting, by the bladder lesion diagnosis system, the unit pathological image to a second neural network to obtain a diagnosis result of a second bladder lesion excluding the first bladder lesion among the plurality of bladder lesions in the unit pathological image,
wherein the first neural network is a neural network trained through a plurality of first training data annotated with a lesion region in which the first bladder lesion is expressed, and
the second neural network is a neural network trained through a plurality of second training data annotated with an expressed lesion type indicating whether at least one of the second bladder lesion is expressed, wherein the second training data is data for which annotation for the lesion region is not performed.

2. The bladder lesion diagnosis method of claim 1, wherein the unit pathological image is a patch image obtained by dividing a pathological image corresponding to a tissue specimen obtained through transurenthral resection of bladder (TURB) into a predetermined size.

3. The bladder lesion diagnosis method of claim 1, wherein the first bladder lesion comprises a urothelial carcinoma in situ (CIS) lesion.

4. The bladder lesion diagnosis method of claim 1, wherein the second bladder lesion comprises at least one of an invasive urothelial carcinoma, a low/high grade noninvasive papillary urothelial carcinoma lesion, a papillary urothelial neoplasm of low malignant potential (PUNLMP) lesion, a urothelial proliferation of unknown malignant potential (UPUMP) lesion, a urothelial papilloma lesion, an inverted urothelial papilloma lesion, and a urothelial dysplasia lesion.

5. A bladder lesion diagnosis method using a neural network, comprising:
receiving, by a bladder lesion diagnosis system, a unit pathological image as input;
inputting, by the bladder lesion diagnosis system, the unit pathological image to a second neural network to obtain a diagnosis result of a second bladder lesion excluding a first bladder lesion among a plurality of bladder lesions in the unit pathological image; and
inputting, by the bladder lesion diagnosis system, the unit pathological image to a first neural network to obtain a diagnosis result of the first bladder lesion among the plurality of bladder lesions in the unit pathological image,
wherein the first neural network is a neural network trained through a plurality of first training data annotated with a lesion region in which the first bladder lesion is expressed, and
the second neural network is a neural network trained through a plurality of second training data annotated with an expressed lesion type indicating whether at least one of the second bladder lesion is expressed, wherein the second training data is data for which annotation for the lesion region is not performed.

6. A computer program stored in a non-transitory computer readable recording medium installed in a data processing device and for performing the method of any one of claims 1 to 5.

7. A bladder lesion diagnosis system using a neural network, comprising:
a processor; and
a memory in which a program executed by the processor is recorded,
wherein the processor is configured to drive the program to:
input a unit pathological image to a first neural network to obtain a diagnosis result of a first bladder lesion among a plurality of bladder lesions in the unit pathological image, and input the unit pathological image to a second neural network to obtain a diagnosis result of a second bladder lesion excluding the first bladder lesion among the plurality of bladder lesions in the unit pathological image, and
wherein the first neural network is a neural network trained through a plurality of first training data annotated with a lesion region in which the first bladder lesion is expressed, and
the second neural network is a neural network trained through a plurality of second training data annotated with an expressed lesion type indicating whether at least one of the second bladder lesion is expressed, wherein the second training data is data for which annotation for the lesion region is not performed.

8. The bladder lesion diagnosis system of claim 7, wherein the first bladder lesion comprises a urothelial carcinoma in situ (CIS) lesion.
